(19) **Europäisches Patentamt**

**European Patent Office**

**Office européen des brevets**

(11) **EP 0 806 199 A2**

(12) **EUROPÄISCHE PATENTANMELDUNG**

(43) Veröffentlichungstag:
12.11.1997 Patentblatt 1997/46

(51) Int. Cl.⁶: **A61K 7/13**

(21) Anmeldenummer: 97106057.9

(22) Anmeldetag: 14.04.1997

(84) Benannte Vertragsstaaten:
**DE ES FR GB IT**

(30) Priorität: **09.05.1996 DE 19618653**

(71) Anmelder: **Wella Aktiengesellschaft**
**64274 Darmstadt (DE)**

(72) Erfinder:
- **Schmitt, Manfred**
**64646 Heppenheim (DE)**
- **Balzer, Wolfgang R., Dr.**
**64665 Alsbach (DE)**
- **Lenz, Uwe, Dr.**
**64673 Zwingenberg (DE)**
- **Niessink, Henk**
**64289 Darmstadt (DE)**

(54) **Mittel und Verfahren zum Färben von Keratinfasern**

(57) Mittel zum Färben von Keratinfasern auf der Basis von Pflanzenfarbstoffen, welches in Form einer Färbemittelsuspension vorliegt, die (a) mindestens einen pulverförmigen Pflanzenfarbstoff, (b) mindestens ein Öl und (c) mindestens einen direktziehenden Farbstoff enthält, sowie Verfahren zum Färben von Haaren unter Verwendung dieses Mittels.

EP 0 806 199 A2

# Beschreibung

Gegenstand der vorliegenden Erfindung ist ein Mittel zum Färben von Keratinfasern auf der Basis von Pflanzenfarbstoffen und direktziehenden Farbstoffen sowie ein Verfahren zum Färben von Haaren unter Verwendung dieses Mittels.

Zum Färben von Keratinfasern, insbesondere menschlichen Haaren, werden in zunehmendem Maße Pflanzenfarben verwendet, da diese Farbstoffe eine schonende Färbung der Keratinfasern ermöglichen.

Die Pflanzenfarben werden hierbei in Form eines Pulvers oder Granulates verwendet, das vor der Anwendung mit Wasser vermischt wird.

Die Verwendung derartiger Pflanzenfarbpulver beziehungsweise Pflanzenfarbgranulate, welche aus mehreren Bestandteilen zusammengesetzt sein können, bringt jedoch eine Vielzahl von Nachteilen mit sich.

So kommt es bei Pflanzenfarbpulvern oder aus mehreren Granulaten zusammengesetzten Pflanzenfarbgranulat-mischungen durch die Verwendung von Rohstoffen mit unterschiedlicher Dichte häufig während des Transportes oder der Lagerung zur Trennung der unterschiedlichen Bestandteile, wobei sich die schweren Bestandteile im unteren Teil und die leichteren Bestandteile im oberen Teil des Behältnisses ansammeln. Diese Entmischung hat zur Folge, daß die gleiche Pulver- oder Granulatmenge je nach ihrem Entnahmeort eine unterschiedliche Zusammensetzung hat und somit auch unterschiedliche Färbeergebnisse aufweisen kann.

Um dieser Entmischung entgegenzuwirken, ist es erforderlich, vor jeder Entnahme das Pulver oder Granulat gründlich zu schütteln, was der Verwender in der Regel jedoch nicht tut.

Eine Entmischung kann auch durch den Einsatz von Pulvergemischen mit sehr kleiner Korngröße verhindert werden. Dies hat jedoch den Nachteil, daß solche Pulvergemische - insbesondere beim Öffnen der Behältnisse, bei der Entnahme des Pulvers oder beim Vermischen mit Wasser - zu einer starken Staubentwicklung neigen.

Weiterhin kommen zum Beispiel Pflanzengranulate zum Einsatz, welche zwar als staubarm gelten, je nach Granulierungsmethode entstehen jedoch mehr oder weniger kompakte Granulate, welche durch die Reibung aneinander, beispielsweise während des Transportes, Feinstaub bilden.

Die mit den vorgenannten Pulvern und Granulaten erhaltenen anwendungsfertigen Pflanzenfarbbreie haben zudem anwendungstechnische Nachteile. So sind sie schlecht aufzutragen und haften nicht genügend am Haar. Während der Einwirkzeit trocknet die Masse aus und bröckelt ab. Ebenso besitzen diese Pflanzenbreie eine schlechte Ausspülbarkeit.

Es wurden bereits zahlreiche Versuche unternommen, dieses Problem zu lösen.

So ist es zum Beispiel aus der EP-OS 0 630 643 bekannt, durch ein spezielles Granulierverfahren unter Zusatz von Wachsen mit einem Fließpunkt von 40 bis 130°C ein rieselfähiges Pulver herzustellen-und damit das Stauben zu minimieren. Ein Stauben dieser Mittel ist aber insbesondere beim Abfüllen des Farbpulvers in eine Anrührschale und bei der Anmischung mit Wasser nicht zur Gänze zu verhindern. Weiterhin ist die Anmischbarkeit mit Wasser je nach dem verwendeten Wachs sehr unterschiedlich und häufig unbefriedigend.

Ein weiteres Problem bei Pflanzenhaarfarben ist die geringe Deckkraft dieser Färbemittel.

Es wurden bereits zahlreiche Versuche zur Lösung dises Problems unternommen, eine befriedigende Lösung konnte jedoch bisher nicht erzielt werden.

Es bestand daher die Aufgabe, eine Pflanzenfarbe zur Verfügung zu stellen, welche die vorstehend beschriebenen Nachteile nicht aufweist, insbesondere staubfrei ist und gleichzeitig eine gute Anmischbarkeit sowie gute anwendungstechnische Eigenschatten, insbesondere bezüglich der Deckkraft und Intensität der Färbungen, aufweist.

Überraschenderweise wurde nunmehr gefunden, daß die vorstehend beschriebene Aufgabenstellung durch eine suspensions-, emulsions- oder dispersionsförmige Pflanzenfarbe, welche zusätzlich direktziehende Farbstoffe enthält, gelöst werden kann.

Gegenstand der vorliegenden Erfindung ist daher ein Mittel zum Färben von Keratinfasern, insbesondere von Haaren, auf der Basis von Pflanzenfarbstoffen, welches dadurch gekennzeichnet ist, daß es in Form einer Färbemittelsuspension (die auch als Färbemitteldispersion oder Färbemittelemulsion bezeichnet werden kann) vorliegt, welche (a) mindestens einen pulverförmigen Pflanzenfarbstoff (b) mindestens ein Öl und (c) mindestens einen direktziehenden Farbstoff enthält.

Als Pflanzenfarbstoff werden gemahlene und/oder gepulverte färbende Pflanzenbestandteile, beispielsweise Hennablätter, Indigoblätter, Kamillenblüten, Curcuma-Wurzeln, Rhabarber, Faulbaumrinde, Olivenblätter, kanadische Blutwurzel, Gelbwurzel, Gelbholz, Rotholz, Rotsandelholz, Blauholz, Krappwurzel, Schwarzer Holunder oder Schwarze Apfelbeere, verwendet, wobei die Pflanzenfarbstoffe in der Färbemittelsuspension in einer Gesamtmenge von 35 bis 75 Gewichtsprozent, insbesondere 40 bis 60 Gewichtsprozent, enthalten sind.

Als Öl eignen sich insbesondere flüssige Silikone oder Paraffine, pflanzliche oder tierische Öle, flüssige Fettsäuren, flüssige Fettsäureester, flüssige alkoxylierte Fettsäureester, flüssige Glyceride, flüssige alkoxylierte Glyceride, flüssige Fettalkohole, flüssige alkoxylierte Fettalkohole, insbesondere flüssige Fettalkoholethoxylate mit 2 bis 5 Ethylenoxideinheiten im Molekül, flüssige mehrwertige Alkohole, flüssige Nonylphenolether, flüssige Polyethylenglykole oder flüssige Polypropylenglykole.

Beispiele für geeignete Öle, welche bei Raumtemperatur (25 °C) flüssig beziehungsweise fließfähig sind, sind Paraffinum perliquidum, Paraffinum subliquidum, Dimethicone, Cyclomethicone, Glycerin, Isopropylpalmitat, Hexyllaurat, Dibutyladipat, Octylpalmitat, Polyethylenglykol(7)glycerylcocoat, Isopropylmyristat, Isopropylstearat, Trioxyethylenlaurylether, Dioxyethylenlaurylether, Ölsäure, Trilaurin, Oleylalkohol, Sonnenblumenöl, Spermacetiöl, Olivenöl und Rizinusöl, wobei die Verwendung von Isopropylpalmitat, Sonnenblumenöl, Olivenöl und Rizinusöl besonders bevorzugt ist.

Das Öl wird, bezogen auf die Gesamtmenge der Färbemittelsuspension, in einer Gesamtmenge von 25 bis 65 Gewichtsprozent, vorzugsweise 35 bis 60 Gewichtsprozent, eingesetzt.

Vorzugsweise ist das erfindungsgemäße Mittel frei von Wachsen; es ist jedoch möglich, geringe Mengen (maximal 5 Gewichtsprozent) an Wachsen zuzusetzen, wenn hierdurch die cremeförmige Konsistenz der Färbemittelemulsion nicht beeinträchtigt wird.

Als direktziehende Farbstoffe können übliche, physiologisch unbedenkliche, direktziehende Farbstoffe aus der Gruppe der Nitrofarbstoffe, Azofarbstoffe, Chinonfarbstoffe und Triphenylmethanfarbstoffe, wie zum Beispiel 3-[(4'-Amino-2'-chlor-5'-nitrophenyl)amino]-1,2-propandiol, 3,3'-[(2''-Chlor-5''-nitro-1'',4''-phenylen)-diamino]-bis-1,2-propandiol, 2-(4'-Amino-3'-nitroanilino)-ethanol, 4,N-Ethyl,N-(2'-hydroxyethyl)amino-1-(2''-hydroxyethyl)amino-2-nitro-benzol, 1-Amino-3-methyl-4-(2'-hydroxyethyl)amino-6-nitro-benzol, 1-(2'-Hydroxyethyl)amino-2-nitro-4-bis-(2''-hydroxyethyl)amino-benzol, 4-Bis-(2'-Hydroxyethyl)amino-1-(2''-methoxyethyl)amino-nitrobenzol, 1-(2',3'-Dihydroxypropyl)amino-2-nitro-4-[methyl-(2''-hydroxyethyl)amino]-benzol, 1-[(2',3'-Dihydroxypropyl)amino]-2-nitro-4-[ethyl-2''-(hydroxyethyl)amino]-benzol, 1-(3'-Hydroxypropylamino)-2-nitro-4-bis-(2''-hydroxyethylamino)-benzol, 1-Amino-4-(2'-hydroxyethyl)-amino-nitrobenzol, 1-Hydroxy-2-amino-4,6-dinitro-benzol, 1-Amino-2-nitro-4-bis-(2'-hydroxyethyl)amino-benzol, 1-Amino-2-nitro-4-(2'-hydroxyethyl)amino-5-chlorbenzol, 1-(2'-Hydroxyethyl)amino-2-nitro-4-amino-benzol, 1-Hydroxy-3-nitro-4-amino-benzol, 1-(2'-Aminoethyl)amino-2-nitro-4-(2''-hydroxyethyl)-oxybenzol, 3-Nitro-4-(2'-hydroxyethyl)amino-phenylglycerinether, 1-Amino-5-chlor-4-(2',3'-dihydroxypropyl)amino-2-nitro-benzol, 1,4-Bis-[(2',3'-dihydroxypropyl)amino]-5-chlor-2-nitro-benzol, 1-Hydroxy-2-(2'hydroxyethyl)amino-4,6-dinitro-benzol, 2-Amino-6-chlor-4-nitrophenol, 1-Hydroxy-3-nitro-4-(3'-hydroxypropylamino)-benzol, 3-Nitro-4-ethylamino-benzoesäure, 4-Amino-2-nitro-diphenylamino-2-carbonsäure, 2-Chlor-6-ethylamino-4-nitrophenol, 2,5-Diamino-6-nitropyridin, 1,2,3,4-Tetrahydro-6-nitrochinoxalin, 4-(2'-Hydroxyethyl)amino-3-nitro-benzonitril, 4-(2'-Hydroxyethyl)amino-3-nitro-benzamid, 1-Amino-2-(2'-hydroxyethyl)amino-5-nitro-benzol, 1-Methoxy-2-(2'-hydroxyethyl)amino-5-nitro-benzol, 1-Hydroxy-3-nitro-4-(2'-hydroxyethyl)amino-benzol, 1-Hydroxy-2-amino-3-nitro-benzol, 1-Amino-2-methyl-6-nitro-benzol, 1-(2'Hydroxyethyl)-oxy-3-methylamino-4-nitro-benzol, 1-Methylamino-2-nitro-5-(2',3'-dihydroxypropyl)-oxybenzol, 1-(2'-Hydroxyethyl)amino-2-hydroxy-4-nitro-benzol, 1-Methoxy-3-(2'-aminoethyl)-amino-4-nitro-benzol, 1-(2'-Ureidoethyl)amino-4-nitro-benzol, 1-(2'-Hydroxyethyl)amino-2-nitro-benzol, 4-(2'-Hydroxyethyl)amino-3-nitro-trifluormethyl-benzol, 2,4-Bis-[N-(2'-hydroxyethyl)amino]-5-chlor-nitrobenzol, 4-(2',3'-Dihydroxypropyl)amino-3-nitro-trifluormethyl-benzol, 4-(2'-Hydroxymethyl)amino-3-nitro-methylbenzol, 4-(2'-Hydroxyethyl)amino-3-nitro-chlorbenzol, 1-(4'-Nitrophenylazo)-2-methyl-4-bis-(2''-hydroxyethyl)amino-benzol, 1-(3'-Nitro-4-amino)-phenylazo-2-hydroxy-7-trimethyl-ammoniumchlorid-naphthalin, 1-(2'Hydroxy-4'sulfo-6'nitro)-naphthylazo-2-hydroxynaphthalin, 1-(4'-Aminophenylazo)-2-methyl-4-bis-[(2'-hydroxyethyl)-amino]-benzol, 5-(4'-Dimethylaminophenylazo)-1,4-dimethyl-triazoniumchlorid, 1-(2'-Methoxyphenylazo)-2-hydroxy-7-trimethylammonium-naphthalinchorid, 1-(4'-Aminophenylazo)-2-hydroxy-7-trimethylammonium-naphthalin, 4-(3'-Trimethylammoniumphenylazo)-N-phenyl-3-methyl-pyrazolon (5), 4-Hydroxy-3-[(4'-sulfo-1'-naphthyl)azo]-1-naphthalinsulfonsäure, 1-(4'-Sulfophenylazo)-2-hydroxynaphthalin, 1-(4'-Sulfonphenylazo)-2-hydroxy-6-sulfo-naphthalin, 4-Amino-[4'-bis-(2''-hydroxyethyl)amino]-azobenzol, 4-Amino-[4'-bis-(2''-hydroxyethyl)amino]-2'-methyl-azobenzol, 3-(2',6'-Diaminopyridyl-3'-azo)-pyridin, 7-Phenylazo-1-amino-3,6-disulfo-8-hydroxy-naphthalin, 5-Acetylamino-4-hydroxy-3-[(2'-methylphenyl)azo]-2,7-naphthalindisulfonsäure, 2-(2',4'-Dimethylphenylazo)-6-(4''-sulfophenylazo)-1,3-dihydroxybenzol, 1,4-Bis-(2',3'-dihydroxypropyl)amino-anthrachinon, 1-Methylamino-4-(2'-hydroxyethyl)amino-anthrachinon, 2-(2'-Aminoethyl)amino-anthrachinon, 2-Brom-4,8-diamino-6-(3'-trimethylammonium)-phenylamino-1,5-naphthochinon, 1-(2'-Sulfo-4'-methyl-phenyl)-amino-4-hydroxy-anthrachinon, 1,4-Diamino-anthrachinon, 1-Amino-2-sulfo-4-cyclohexylamino-anthrachinon, 1-Methylamino-4-aminopropylamino-anthrachinon, 1-Aminopropylamino-anthrachinon, 4',4'',4'''-Triamino-3-methyl-triphenylcarboniumchlorid, Bis-(4,4-Diethylaminophenyl)-4'-ethylamino-naphthyl-carboniumchlorid, Bis-(4,4-Dimethylaminophen)-4'-phenylamino-naphthyl-carboniumchlorid und 4,4-Bis-(N-Ethyl-3-sulfobenzyl)-amino-2''-sulfofuchsonium sowie aus natürlichen Stoffen gewonnene Farbstoffe, wie zum Beispiel Lawson oder Yuglon, verwendet werden, wobei diese Farbstoffe in einer Gesamtmenge von etwa 0,001 bis 15 Gewichtsprozent, vorzugsweise 0,3 bis 10 Gewichtsprozent, eingesetzt werden können.

Zusätzlich kann die cremeförmige Färbemittelsuspension weitere für derartige Zusammensetzungen übliche Zusatzstoffe, beispielsweise Parfümöle, färbende tierische Bestandteile wie Cochenille oder Lac Dye, nicht-färbende pflanzliche Bestandteile wie getrocknete und gepulverte Akazienblätter, Lindenblätter, Birkenblätter, Weizen, Roggen, Gerste, Süßholz, Catechu oder Salbeikraut, Verdicker wie Cellulosen, Alginate, Polysaccharide oder mineralische Verdicker wie Bentonit, Tenside und Emulgatoren, Antioxidantien wie Butylhydroxyanisol, Butylhydroxytoluol oder Tocopherol, Fruchtextrakte, Chelatisierungsmittel oder Lösungsvermittler wie Polyethylenglykole, beispielsweise Polyethylenglykol(4), enthalten.

Als Verdicker aus der Gruppe der Cellulosen, Alginate und Polysaccharide werden vorzugsweise Methylcellulosen, Ethylcellulosen, Hydroxyethylcellulosen, Mehtylhydroxyethylcellulosen, Methylhydroxypropylcellulosen, Carboxymethylcellulosen, Alginsäure, Natriumalginat, Ammoniumalginat, Calciumalginat, Gummi Arabicum, Guar Gum oder Xanthan Gum, sowie deren kationische Derivate, alleine oder in Kombination verwendet, wobei die Einsatzmenge, bezogen auf die Gesamtmenge der Färbemittelsuspension, 0,1 bis 15 Gewichtsprozent, vorzugsweise 0,2 bis 10 Gewichtsprozent, beträgt.

Als Tenside und Emulgatoren können nichtionische, kationische, anionische oder amphotere oberflächenaktive Verbindungen und O/W- oder W/O-Emulgatoren, wie zum Beispiel Silikontenside, Alkyletherphosphate, Glyceridalkoxylate, Alkylsulfate, Alkalimetallsalze oder Erdalkalimetallsalze von Fettsäuren, Alkylethersulfate, Alkylsulfoacetate, oxethylierte Fettsäureester und Fettalkoholalkoxylate, verwendet werden, wobei die Einsatzmenge dieser Verbindungen, bezogen auf die Gesamtmenge der Färbemittelsuspension, 0,1 bis 20 Gewichtsprozent, vorzugsweise 0,5 bis 10 Gewichtsprozent, beträgt.

Weiterhin kann die erfindungsgemäße Färbemittelsuspension zur Einstellung des gewünschten pH-Wertes Akalisierungsmittel, wie zum Beispiel Alkalihydroxide, Natriumcarbonat, Natriumhydrogencarbonat, Magnesiumcarbonat, Ammoniumcarbonat, Ammoniumhydrogencarbonat oder Natriumsilikate, oder Ansäuerungsmittel, wie zum Beispiel Zitronensäure, Weinsäure, Ammoniumchlorid oder Ammoniumsulfat, enthalten.

Vor der Anwendung wird die cremeförmige Färbemittelsuspension mit kaltem oder warmem (T = 10 bis 90 °C) Wasser zu einem auftragefähigen Färbebrei vermischt, wobei dieses Vermischen in einer Schale oder durch Anschütteln in einer Auftrageflasche erfolgen kann.

Das Mischungsverhältnis von Färbemittelsuspension zu Wasser beträgt vorzugsweise 10 : 1 bis 1 : 10.

Das so erhaltene gebrauchsfertige Mittel zum Färben von Haaren ("Färbebrei") wird auf das Haar gleichmäßig aufgetragen und nach einer Einwirkungszeit von 5 bis 80 Minuten, vorzugsweise 15 bis 60 Minuten bei Raumtemperatur (20 bis 25 °C) beziehungsweise 10 bis 50 Minuten unter Wärmeeinwirkung (30 bis 50 °C), mit Wasser ausgespült und getrocknet. Es ist jedoch auch möglich, die Färbemittelsuspension direkt auf das vorher mit Wasser angefeuchtete Haar aufzutragen.

Die cremeförmige Färbemittelsuspension kann je nach ihrer Viskosität in Tuben oder Tiegeln abgefüllt werden, ist staubfrei und zeichnet sich durch eine gute Anmischbarkeit, Auftragefähigkeit, Verteilbarkeit und Haftung am Haar sowie hervorragende Färbeeigenschaften aus.

Die nachfolgenden Beispiele sollen den Gegenstand näher erläutern, ohne diesen hierauf zu beschränken.

**Beispiele**

**Beispiel 1:** Cremeförmige Färbemittelsuspension

| | |
|---|---|
| 45,0 g | Isopropylpalmitat |
| 38,5 g | Hennablätter, getrocknet und gemahlen |
| 10,0 g | Natriumcarbonat |
| 3,0 g | Polyethylenglykol(35)rizinusöl (Cremophor EL der Firma BASF) |
| 1,5 g | kationischer Guar Gum |
| 1,4 g | 3-[(4'-Amino-2'-chlor-5'-nitrophenyl)amino]-1,2-propandiol (HC Red No. 10) |
| 0,6 g | 3,3'-[(2''-Chlor-5''-nitro-1',4''-phenylen)-diamino]-bis-1,2-propandiol (HC Red No. 11) |
| 100,00 g | |

25 g der vorstehend beschriebenen Färbemittelsuspension werden mit 75 g warmem (T = 40 °C) Wasser in einer Schale mit einem Pinsel oder Schneebesen zu einem homogenen Färbebrei verrührt. Der so erhaltene Färbebrei wird mit einem Pinsel auf hellblonde Haare gleichmäßig aufgetragen. Anschließend wird das Haar mit einer Folie abgedeckt und 40 Minuten lang mit einer Trockenhaube oder einem Wärmestrahler auf 40 °C erwärmt. Sodann werden die Haare mit warmem Wasser gründlich ausgespült und getrocknet.

Als Ergebnis dieser Färbebehandlung wird eine intensive rotorangeblonde Färbung der Haare erhalten.

**Beispiel 2:** Cremeförmige Färbemittelsuspension

| | |
|---|---|
| 43,0 g | Rizinusöl |
| 38,5 g | Hennablätter, getrocknet und gemahlen |
| 10,0 g | Natriumhydrogencarbonat |
| 5,0 g | Tetraoxyethylenlaurylether-triphosphat (Hostaphat KL 340 N der Firma Hoechst-Celanese Corp.) |
| 1,5 g | Natriumalginat |
| 1,0 g | 2-(4'-Amino-3'-nitroanilino)-ethanol (HC Red No. 12) |
| 0,7 g | 3-[(4'-Amino-2'-chlor-5'-nitrophenyl)amino]-1,2-propandiol (HC Red No. 10) |
| 0,3 g | 3,3'-[(2''-Chlor-5''-nitro-1',4''-phenylen)-diamino]-bis-1,2-propandiol (HC Red No. 11) |
| 100,00 g | |

30 g der vorstehend beschriebenen Färbemittelsuspension werden mit 60 g heißem (T = 70 °C) Wasser in einer Schale mit einem Pinsel oder einem Schneebesen zu einem homogenen Färbebrei verrührt. Der so erhaltene Färbebrei wird mit einem Pinsel auf dunkelblonde Haare (50 % Grauanteil) gleichmäßig aufgetragen. Anschließend wird das Haar mit einer Folie abgedeckt und 40 Minuten lang mit einer Trockenhaube oder einem Wärmestrahler auf 40 °C erwärmt. Sodann werden die Haare mit warmem Wasser gründlich ausgespült und getrocknet.

Es wird eine intensive blaurote Färbung erhalten, mit einer Deckkraft von etwa 30 %.

Alle Prozentangaben stellen, soweit nicht anders angegeben, Gewichtsprozente dar.

**Patentansprüche**

1. Mittel zum Färben von Keratinfasern auf der Basis von Pflanzenfarbstoffen, dadurch gekennzeichnet, daß es in Form einer Färbemittelsuspension vorliegt, welche (a) mindestens einen pulverförmigen Pflanzenfarbstoff, (b) mindestens ein Öl und (c) mindestens einen direktziehenden Farbstoff enthält.

2. Mittel nach Anspruch 1, dadurch gekennzeichnet, daß es eine anionische, kationische, nichtionische und/oder amphotere oberflächenaktive Verbindung enthält.

3. Mittel nach Anspruch 1 oder 2, dadurch gekennzeichnet, daß der pulverförmige Pflanzenfarbstoff ausgewählt ist aus Hennablättern, Indigoblättern, Kamillenblüten, Curcuma-Wurzeln, Rhabarber, Faulbaumrinde, Olivenblättern, kanadische Blutwurzel, Gelbwurzel, Gelbholz, Rotholz, Rotsandelholz, Blauholz, Krappwurzel, Schwarzer Holunder und Schwarze Apfelbeere.

4. Mittel nach einem der Ansprüche 1 bis 3, dadurch gekennzeichnet, daß der Pflanzenfarbstoff in der Färbemittelsuspension in einer Gesamtmenge von 35 bis 75 Gewichtsprozent enthalten ist.

5. Mittel nach einem der Ansprüche 1 bis 4, dadurch gekennzeichnet, daß das Öl ausgewählt ist aus flüssigen Silikonen oder Paraffinen, pflanzlichen oder tierischen Ölen, flüssigen Fettsäuren, flüssigen Fettsäureester, flüssigen alkoxylierten Fettsäureestern, flüssigen Glyceriden, flüssigen alkoxylierten Glyceriden, flüssigen Fettalkoholen, flüssigen alkoxylierten Fettalkoholen, flüssigen mehrwertigen Alkoholen, flüssigne Nonylphenolether, flüssigen Polyethylenglykolen oder flüssigen Polypropylenglykolen.

6. Mittel nach einem der Ansprüche 1 bis 5, dadurch gekennzeichnet, daß das Öl ausgewählt ist aus Paraffinum perliquidum, Paraffinum subliquidum, Dimethicone, Cyclomethicone, Glycerin, Isopropylpalmitat, Hexyllaurat, Dibutyladipat, Octylpalmitat, Isopropylmyristat, Isopropylstearat, Trioxyethylenlaurylether, Dioxyethylenlaurylether, Trilaurin, Oleylalkohol, Ölsäure, Polyethylenglykol(7)-glycerylcocoat, Sonnenblumenöl, Olivenöl, Spermacetiöl und Rizinusöl.

7. Mittel nach einem der Ansprüche 1 bis 6, dadurch gekennzeichnet, daß das Öl in der Färbemittelsuspension in einer Gesamtmenge von 25 bis 65 Gewichtsprozent enthalten ist.

8. Mittel nach einem der Ansprüche 1 bis 7, dadurch gekennzeichnet, daß der direktziehende Farbstoff ausgewählt ist aus Nitrofarbstoffen, Azofarbstoffen, Chinonfarbstoffen, Triphenylmethan-farbstoffen und aus natürlichen Stoffen gewonnenen Farbstoffen.

9. Mittel nach einem der Ansprüche 1 bis 8, dadurch gekennzeichnet, daß der direktziehende Farbstoff in der Färbemittelsuspension in einer Gesamtmenge von 0,001 bis 15 Gewichtsprozent enthalten ist.

10. Verfahren zum Färben von Haaren, dadurch gekennzeichnet, daß das Haar mit Wasser angefeuchtet wird, sodann eine Färbemittelsuspension gemäß einem der Ansprüche 1 bis 9 auf das Haar aufgetragen wird und nach einer Einwirkungszeit von 5 bis 80 Minuten das Haar mit Wasser ausgespült und getrocknet wird.

11. Verfahren zum Färben von Haaren, dadurch gekennzeichnet, daß eine Färbemittelsuspension gemäß einem der Ansprüche 1 bis 9 mit Wasser im Verhältnis 10 : 1 bis 1 : 10 zu einem Färbebrei vermischt wird, anschließend dieser Färbebrei gleichmäßig auf das Haar aufgetragen wird und nach einer Einwirkungszeit von 5 bis 80 Minuten das Haar mit Wasser ausgespült und getrocknet wird.

12. Mittel nach Anspruch 10 oder 11, dadurch gekennzeichnet, daß die Einwirkungszeit 15 bis 60 Minuten (bei Raumtemperatur) oder 10 bis 50 Minuten (unter Wärmeeinwirkung) beträgt.